# EUROPÄISCHE PATENTSCHRIFT

(11) **EP 0 552 748 B1**
(45) Veröffentlichungstag und Bekanntmachung des Hinweises auf die Patenterteilung: **02.12.1998**
(21) Anmeldenummer: 93100825.4
(22) Anmeldetag: 20.01.1993
(51) Int. Cl.: A61F 13/15

(54) **Flüssigkeitsdurchlässige Decklage für Körperflüssigkeit absorbierende Artikel**
Permeable topsheet for body-fluid absorbing article
Feuille supérieure perméable pour article absorbant des liquides corporels

(30) Priorität: 20.01.1992 JP 1540/92
(43) Veröffentlichungstag der Anmeldung: 28.07.1993
(73) Patentinhaber: UNI-CHARM CORPORATION, Kawanoe-shi Ehime-ken (JP)
(72) Erfinder: Murakami, Masaki, Kawanoe-shi, Ehime-ken (JP); Inagaki, Hiroyuki, Kakegawa-shi, Shizuoka-ken (JP); Yamada, Yozo, Kakegawa-shi, Shizuoka-ken (JP)
(74) Vertreter: Sperling, Rüdiger, Dipl.-Ing.

(56) Entgegenhaltungen:
- EP-A- 0 018 020
- EP-A- 0 039 974
- EP-A- 0 305 123
- EP-A- 0 313 766
- WO-A-90/11746
- GB-A- 2 235 878
- US-A- 2 896 618
- US-A- 3 459 618

## Beschreibung

Diese Erfindung betrifft eine flüssigkeitsdurchlässige Decklage aus einem thermoplastischen Kunststoff, die für Körperflüssigkeit absorbierende Artikel, wie z.B. Wegwerfwindeln und Damenbinden, verwendet wird, nach dem Oberbegriff des Anspruchs 1.

Herkömmlicherweise wurden Vliesstoff, perforierte thermoplastische Folie oder ähnliches als flüssigkeitsdurchlässige Decklage verwendet, die einen Absorptionskern von Körperflüssigkeit absorbierenden Artikeln bedeckt.

Es wird offensichtlich angestrebt, daß die Körperflüssigkeitsabsorption derartiger Artikel so schnell wie möglich erfolgen sollte. Dies wird beispielsweise dadurch in wirksamer Weise erzielt, daß der Fluß der Körperflüssigkeit auf einer Decklage ausgerichtet wird, oder durch die Steuerung der Diffusion der Körperflüssigkeit in einer vorgegebenen Richtung, so daß sich die Körperflüssigkeit rasch über die gesamte Oberfläche des Absorptionskerns verteilen kann. Während eine aus Vliesstoff hergestellte Decklage insofern zu bevorzugen ist, als ein bestimmter Diffusionsgrad unter einem zwischen den den Vliesstoff bildenden Fasern und der Körperflüssigkeit entstehenden Kapillareffekt erwartet werden kann, ist es problematisch, die rasche und richtungsgesteuerte Diffusion der Körperflüssigkeit zu verwirklichen, da die Dichte des Vliesstoffes für eine derartige Anwendung durch das Erfordernis beschränkt ist, daß der Griff der Decklage für die Haut des Benutzers weich sein sollte. Wenn als Decklage eine perforierte Folie verwendet wird, so fließt andererseits die Körperflüssigkeit gleichmäßig auf der Oberfläche der Folie, wobei jedoch keine Richtungssteuerung möglich ist.

Aus der WO-A-90 117 46 ist für einen Tampon eine flüssigkeitsdurchlässige Decklage aus thermoplastischem Kunststoff bekannt, mit einer Vielzahl von Rippen, die in einer ersten Richtung verlaufen, und einer Vielzahl von Rippen, die in einer zweiten Richtung verlaufen, die jeweils eine in ihrer Längsrichtung verlaufende Oberseite aufweisen, wobei die Rippen erster Richtung und die Rippen zweiter Richtung einander überkreuzen und jeweils zwei benachbarte Rippen erster Richtung und jeweils zwei benachbarte Rippen zweiter Richtung, die die beiden benachbarten Rippen erster Richtung überkreuzen, jeweils eine flüssigkeitsdurchlässige Öffnung bilden. Die Rippen verlaufen jeweils schräg zur Hauptachse des Tampons, um diesen leichter und angenehmer einzuführen zu können. Eine Steuerung des Flusses von Körperflüssigkeit wird dadurch weder angestrebt noch realisiert.

Die US-A-2 896 618 betrifft einen gewellten Verband, der aus einer gewellten flüssigkeitsabsorbierenden Kompresse und einem diese beidseitig abdeckenden dünnen, nicht wasserlöslichen, perforierten, flexiblen, glatten Film besteht. Der Film liegt mit seiner Unterseite nur auf den Rücken der Hauptwulste auf, die höher als quer dazu verlaufende Zusatzwulste sind so daß die glatte Filmoberfläche an der Stelle anliegt, an der Flüssigkeit durch den Film hindurch in die absorbierende Kompresse abgeleitet werden soll.

Die Aufgabe der vorliegenden Erfindung besteht darin, die Decklage derart auszugestalten, daß eine Steuerung des Flusses von Körperflüssigkeit in eine vorgegebene Richtung ermöglicht wird.

Die Lösung dieser Aufgabe besteht in einer Decklage nach dem Anspruch 1. Bevorzugte Weiterbildungen sind in den auf den Anspruch 1 rückbezogenen Ansprüchen angegeben.

### BESCHREIBUNG DER ERFINDUNG

Gemäß der Erfindung wird eine aus thermoplastischem Kunststoff hergestellte flüssigkeitsdurchlässige Decklage für Körperflüssigkeit absorbierende Artikel geschaffen wobei die flüssigkeitsdurchlässige Decklage eine Vielzahl von Rippen in einer ersten Richtung umfaßt, die jeweils eine in Längsrichtung in einer ersten Richtung verlaufende Oberseite sowie gegenüberliegende Seiten umfassen, die von dieser Oberseite gekrümmt abwärts verlaufen und sich in Längsrichtung ebenfalls in die erste Richtung erstrecken, sowie eine Vielzahl von Rippen in einer zweiten Richtung, die jeweils mit einer Oberseite versehen sind, die sich in Längsrichtung in eine zweite Richtung erstreckt, wobei diese Rippen der ersten und zweiten Richtung einander überkreuzen. Jeweils zwei benachbarte Rippen erster Richtung und jeweils zwei benachbarte Rippen zweiter Richtung, die die beiden benachbarten Rippen erster Richtung kreuzen, bilden jeweils eine flüssigkeitsdurchlässige Öffnung. An den jeweiligen Kreuzungspunkten dieser Rippen erster Richtung und zweiter Richtung sind die Oberseiten der Rippen zweiter Richtung mit den Seiten der Rippen erster Richtung verbunden, so daß sie eine Kreuzrippenstruktur bilden, die das wichtigste Merkmal der Erfindung bildet.

Bei der oberen Lage mit einer derartigen Rippenstruktur verlaufen eine Vielzahl von Rippen erster Richtung parallel zueinander in der ersten Richtung und jeweils zwei benachbarte Rippen erster Richtung werden von den Rippen zweiter Richtung in einem vorgegebenen Abstand zueinander beabstandet gehalten. Die Oberseiten der Rippen zweiter Richtung sind mit den Seiten der Rippen erster Richtung verbunden, so daß dadurch jeweils zwei benachbarte Rippen erster Richtung einen in der ersten Richtung verlaufenden Kanal bilden, auf dessen Boden sich die Rippen zweiter Richtung und die Öffnungen befinden Der Kanal erleichtert das Fließen der Körperflüssigkeit eher in der ersten Richtung als in der quer zu dieser verlaufenden Richtung.

### KURZE BESCHREIBUNG DER ZEICHNUNG

Die Erfindung wird detaillierter unter Bezug auf die beiliegende Zeichnung erläutert, wobei
- Fig. 1: eine perspektivische Darstellung der gemäß der Erfindung aufgebauten Decklage ist;
- Fig. 2: eine Schnittdarstellung entlang einer Linie X-X in Fig. 1 ist;
- Fig. 3: eine Fig. 1 ähnliche Ansicht ist, die aber eine weitere Ausführungsform der Erfindung zeigt;
- Fig. 4: eine Fig. 1 ähnliche Ansicht ist, die abermals eine weitere Ausführungsform der Erfindung zeigt; und
- Fig. 5: eine perspektivische Darstellung einer Damenbinde ist.

### BEVORZUGTE AUSFÜHRUNGSFORMEN DER ERFINDUNG

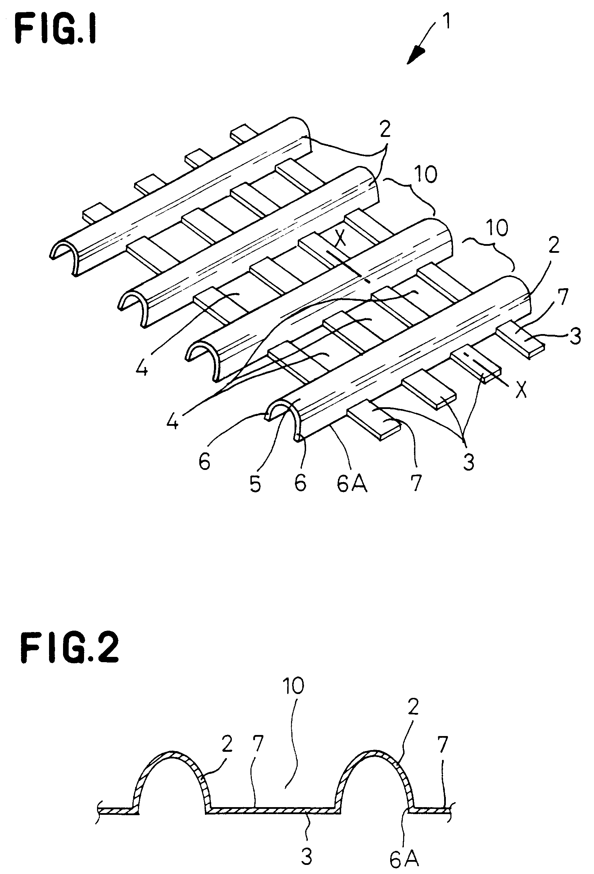

Fig. 1 ist eine perspektivische Darstellung der Oberflächenkonfiguration einer gemäß der Erfindung aufgebauten Decklage 1. Die Decklage 1 umfaßt eine Vielzahl von Rippen 2 erster Richtung und eine Vielzahl von Rippen 3 zweiter Richtung, die die Rippen 2 erster Richtung überkreuzen, wobei jeweils zwei benachbarte Rippen 2 erster Richtung und jeweils 2 benachbarte Rippen 3 zweiter Richtung eine flüssigkeitsdurchlässige Öffnung 4 bilden. Jede Rippe 2 erster Richtung hat eine Oberseite 5, die sich in der ersten Richtung längs erstreckt, sowie gegenüberliegende Seiten 6, die von der Oberseite 5 in einer Krümmung zu jeweiligen unteren Rändern 6A nach unten verlaufen. Die Rippen 3 zweiter Richtung sind jeweils flach und lagenartig und erstrecken sich in Längsrichtung in der zweiten Richtung, wobei sie eine durch ihre Oberfläche gebildete Oberseite 7 besitzen. Die Rippen 3 zweiter Richtung sind mit den Seiten 6 der Rippen 2 erster Richtung entlang deren unteren Rändern 6A oder unmittelbar an diese anschließend an den jeweiligen Kreuzungspunkten der Rippen 2 erster Richtung und der Rippen 3 zweiter Richtung verbunden, wodurch jeweils zwei benachbarte Rippen 2 erster Richtung zueinander in einem vorgegebenen Abstand beabstandet gehalten werden, so daß sie einen in die erste Richtung verlaufenden Kanal 10 bilden. Dieser Kanal 10 umfaßt auf seinem Boden die Rippen 3 zweiter Richtung und die Öffnungen 4, die flüssigkeitsdurchlässig sind.

Fig. 2 ist eine Schnittdarstellung entlang einer Linie X-X in Fig. 1 und zeigt, wie die unteren Ränder 6A der jeweiligen Rippen 2 erster Richtung mit den Oberseiten 7 der jeweiligen Rippen 3 zweiter Richtung verbunden sind und wie der Kanal durch jeweils zwei benachbarte Rippen 2 erster Richtung gebildet wird.

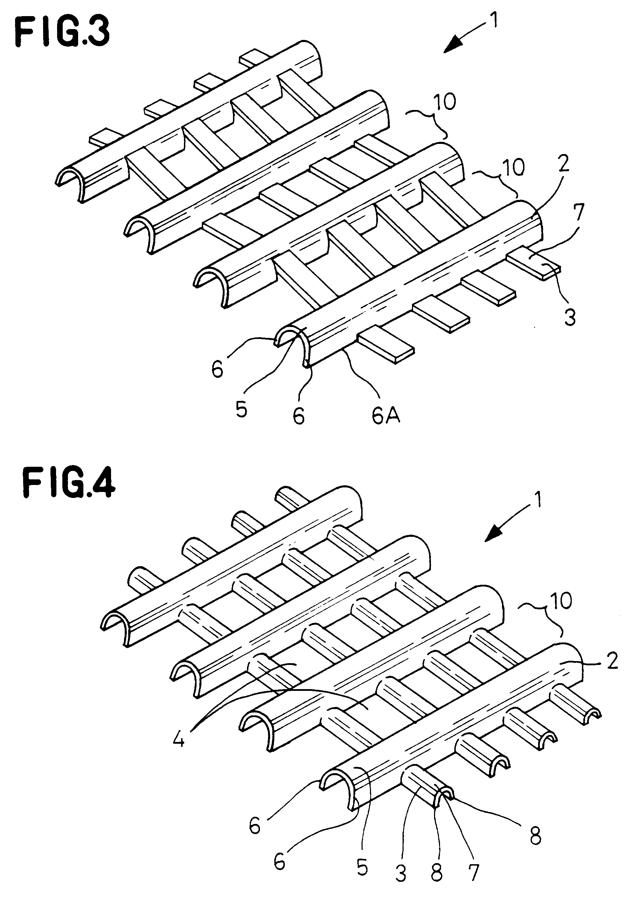

Fig. 3 ist eine perspektivische Darstellung ähnlich Fig. 1, die jedoch eine weitere Ausführungsform der Erfindung zeigt, wobei die Oberseiten 7 der jeweiligen Rippen 3 zweiter Richtung mit einer von zwei benachbarten Rippen 2 erster Richtung unmittelbar am unteren Rand 6A dieser Rippe 2 erster Richtung verbunden sind, aber mit der anderen der beiden benachbarten Rippen 2 erster Richtung im oberen Bereich ihrer Seite 6 verbunden sind. Bei dieser Anordnung wird ebenfalls der Kanal 10 gebildet.

Fig. 4 ist eine perspektivische Darstellung ähnlich Fig. 1, zeigt jedoch eine weitere Ausführungsform der Erfindung, wobei die Rippen 3 zweiter Richtung sowohl hinsichtlich der Breite als auch hinsichtlich der Höhe kleiner als die Rippen 2 erster Richtung sind, jedoch über Oberseiten 7 und gegenüberliegende Seiten 8 verfügen, die ähnlich denen der Rippen 2 erster Richtung sind. Die Oberseiten 7 sind mit den gegenüberliegenden Seiten 6 der Rippen 2 erster Richtung verbunden. Die Oberseiten 7 der Rippen 3 zweiter Richtung ragen nicht über die Oberseiten 5 der Rippen 2 erster Richtung hervor, und jeweils zwei benachbarte Rippen 2 erster Richtung bilden den Kanal 10, der in der ersten Richtung verläuft.

Die Abmessungen der jeweiligen Bestandteile der Decklage 1 sind vorzugsweise so gewählt, daß der Kanal 10 eine Breite von 0,1 bis 3 mm, gemessen zwischen den gegenüberliegenden unteren Rändern 6A eines jeden Paares von benachbarten Rippen 2 erster Richtung, aufweist, die Rippen 2 erster Richtung und Rippen 3 zweiter Richtung Breiten von 0,3 bis 3 mm aufweisen, die Rippen 2 erster Richtung eine Höhe von 0,2 bis 3 mm aufweisen, die Rippen 3 zweiter Richtung eine geringere Höhe als die der Rippen 2 erster Richtung aufweisen, und die Öffnung 4 eine Fläche von 0,1 bis 9 mm² aufweist. Die Rippe 2 erster Richtung hat eine Stärke von 0,02 bis 0,2 mm und die Rippe 3 zweiter Richtung eine Stärke, die gleich oder kleiner ist als die Stärke der Rippe 2 erster Richtung. Diese Decklage 1 kann durch thermisches Formen einer geeigneten thermoplastischen Folie, wie etwa einer Polyethylenfolie erhalten werden. Ist die Decklage 1 hydrophob, so kann die Oberfläche der Decklage 1 einer geeigneten Behandlung mit einem hydrophilen Wirkstoff unterzogen werden, um ihre Benetzbarkeit mit Körperflüssigkeit zu steuern, so daß sich die Körperflüssigkeit in der ersten Richtung unter einem zwischen den Seitenwänden der jeweiligen Kanäle 10 und der Körperflüssigkeit auftretenden Kapillareffekt rasch verteilt.

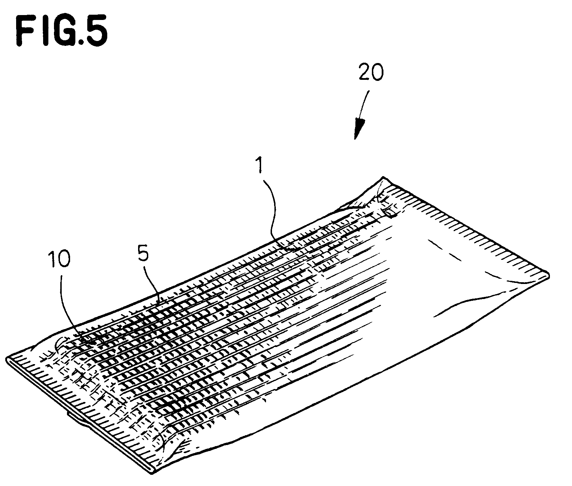

Fig. 5 ist eine perspektivische Darstellung einer Damenbinde 20, für die die Decklage 1 der Erfindung verwendet wird. Die Damenbinde 20 ist in rechtwinkeliger Form zugeschnitten, die in Übereinstimmung mit der Richtung der Kanäle 10 in Längsrichtung verläuft. Bei der Verwendung der Damenbinde kommen die Oberseiten 5 der jeweiligen Rippen 2 erster Richtung mit der Haut der Benutzerin in Berührung. Demzufolge fließt einmal auf die Decklage 1 abgegebene Körperflüssigkeit rasch entlang den jeweiligen Kanälen 10 und verteilt sich zu den in Längsrichtung gegenüberliegenden Enden derselben. Eine quer zur Damenbinde fließende Menge von Körperflüssigkeit wird dementsprechend unterdrückt und das Austreten von Körperflüssigkeit kann an beiden Seiten der Binde 20 wirksam vermieden werden.

Die erfindungsgemäße Decklage erlaubt es, den Fluß der Körperflüssigkeit zu steuern, indem die Decklage mit in einer vorgegebenen Richtung ausgerichteten Kanälen verwendet wird, und erlaubt es gleichzeitig, das Austreten von Körperflüssigkeit in Querrichtung der Kanäle zu verhindern.

## Patentansprüche

1. Flüssigkeitsdurchlässige Decklage aus thermoplastischem Kunststoff für Körperflüssigkeit absorbierende Artikel, mit je einer Vielzahl von Rippen, die in einer ersten und in einer zweiten Richtung verlaufen, wobei die Rippen erster Richtung und die Rippen zweiter Richtung eine Gitterstruktur mit darin enthaltenen flüssigkeitsdurchlässigen Öffnungen bilden und die Rippen zweiter Richtung eine geringere Dicke als die Rippen erster Richtung haben,
**dadurch gekennzeichnet,**
daß die Rippen (2) erster Richtung je eine Oberseite (5) und von dieser in einer Krummung nach unten verlaufende Seiten (6) aufweisen, und
daß die Rippen (3) zweiter Richtung an den Seiten (6) der Rippen (2) erster Richtung so anschließen, daß dadurch jeweils zwei benachbarte Rippen (2) erster Richtung einen in der ersten Richtung verlaufenden Kanal (10) bilden.

2. Flüssigkeitsdurchlässige Decklage nach Anspruch 1, dadurch gekennzeichnet, daß die Rippen (2) erster Richtung rinnenförmig nach unten offen ausgebildet sind.

3. Flüssigkeitsdurchlässige Decklage nach Anspruch 1 oder 2, dadurch gekennzeichnet, daß die Rippen (3) zweiter Richtung rinnenförmig nach unten offen ausgebildet sind.

4. Flüssigkeitsdurchlässige Decklage nach einem der vorhergehenden Ansprüche, dadurch gekennzeichnet, daß die Seiten (6) der Rippen (2) erster Richtung freie untere Ränder (6A) aufweisen, entlang denen die Rippen (3) zweiter Richtung mit ihrer Oberseite (7) mit den Rippen (2) erster Richtung verbunden sind.

5. Flüssigkeitsdurchlässige Decklage nach einem der Ansprüche 1 bis 3, dadurch gekennzeichnet, daß die Rippen (3) zweiter Richtung zwischen zwei benachbarten Rippen (2) erster Richtung von einem freien unteren Rand (6A) der Seite (6) einer der beiden benachbarten Rippen (2) erster Richtung, die der anderen der beiden benachbarten Rippen (2) erster Richtung gegenüber liegt, zum oberen Bereich der gegenüberliegenden Seite (6) der anderen der benachbarten Rippen (2) erster Richtung verlaufen.

6. Flüssigkeitsdurchlässige Decklage nach einem der vorhergehenden Ansprüche, dadurch gekennzeichnet, daß zwischen zwei benachbarten Rippen (2) erster Richtung je ein in dieser ersten Richtung verlaufender Kanal (10) gebildet ist.

7. Flüssigkeitsdurchlässige Decklage nach Anspruch 6, dadurch gekennzeichnet, daß der Kanal (10) auf seinem Boden die Rippen (3) zweiter Richtung und die Öffnungen (4) umfaßt.

8. Flüssigkeitsdurchlässige Decklage nach einem der vorhergehenden Ansprüche, dadurch gekennzeichnet, daß die Rippen (3) zweiter Richtung eine geringere Höhe als die Rippen (2) erster Richtung aufweisen.

9. Flüssigkeitsdurchlässige Decklage nach einem der vorhergehenden Ansprüche, dadurch gekennzeichnet, daß die Decklage (1) durch thermisches Formen einer thermoplastischen Folie gebildet ist

10. Flüssigkeitsdurchlässige Decklage nach einem der vorhergehenden Ansprüche, dadurch gekennzeichnet, daß die Decklage (1) hydrophob ist und mit einem hydrophilen Wirkstoff versehen ist.

## Claims

1. A liquid-permeable topsheet of thermoplastic plastics for body fluid absorbing articles, comprising a plurality of ribs extending in a first direction and a second direction, respectively, the ribs of the first direction and the ribs of the second direction forming a lattice structure containing liquid-permeable openings, the ribs of the second direction having a smaller thickness than the ribs of the first direction,
characterized in that
the ribs (2) of the first direction each have a top face (5) and sidefaces (6) extending therefrom in a downward curve, and
that the ribs (3) of the second direction are connected with the sidefaces (6) of the ribs (2) of the first direction in such a way that each pair of adjacent ribs (2) of the first direction form a channel (10) in the first direction.

2. A liquid-permeable topsheet as claimed in Claim 1, characterized in that the ribs (2) of the first direction have a groove-like, downward open structure.

3. A liquid-permeable topsheet as claimed in Claim 1 or 2, characterized in that the ribs (3) of the second direction have a groove-like, downward open structure.

4. A liquid-permeable topsheet as claimed in one of the preceding Claims, characterized in that the sidefaces (6) of the ribs (2) of the first direction have free lower edges (6A) along which the ribs (3) of the second direction are joined at their upper faces (7) with the ribs (2) of the first direction.

5. A liquid-permeable topsheet as claimed in one of the Claims 1 to 3, characterized in that the ribs (3) of the second direction extend between two adjacent ribs (2) of the first direction from a free lower edge (6A) of the sideface (6) of one of the two adjacent ribs (2) of the first direction facing the other one of the two adjacent ribs (2) of the first direction to the upper region of the opposite sideface (6) of the other one of the two adjacent ribs (2) of the first direction.

6. A liquid-permeable topsheet as claimed in one of the preceding Claims, characterized in that between two adjacent ribs (2) of the first direction one channel (10) extending in this first direction is formed, respectively .

7. A liquid-permeable topsheet as claimed in Claim 6, characterized in that the channel (10) comprises at its bottom the ribs (3) of the second direction and the openings (4).

8. A liquid-permeable topsheet as claimed in one of the preceding Claims, characterized in that the ribs (3) of the second direction have a smaller height than the ribs (2) of the first direction.

9. A liquid-permeable topsheet as claimed in one of the preceding Claims, characterized in that the topsheet (1) is formed by thermal molding of a thermoplastic film.

10. A liquid-permeable topsheet as claimed in one of the preceding Claims, characterized in that the topsheet is hydrophobic and is treated with a hydrophilic agent.

## Revendications

1. Couche de revêtement perméable aux liquides en matière thermoplastique, pour articles absorbant les fluides corporels, comprenant une multiplicité de côtes orientées dans un premier sens et de côtes orientées dans un second sens, couche dans laquelle les côtes de premier sens et les côtes de second sens forment une structure quadrillée avec des ajours laissant passer les liquides compris dans cette structure, et dans laquelle les côtes de second sens présentent une épaisseur inférieure à celle des côtes de premier sens,
caractérisée en ce que :
les côtes (2) de premier sens présentent chacune une face supérieure (5) et des faces (6) s'incurvant vers le bas en partant de cette dernière, et
en ce que les côtes (3) de second sens se rattachent aux faces (6) des côtes (2) de premier sens, de telle manière que deux côtes voisines (2) de premier sens forment chaque fois un canal (10) s'étendant dans le premier sens.

2. Couche de revêtement perméable aux liquides selon la revendication 1, caractérisée en ce que les côtes (2) de premier sens sont configurées à la manière de rigoles ouvertes vers le bas.

3. Couche de revêtement perméable aux liquides selon l'une des revendications 1 ou 2, caractérisée en ce que les côtes (3) de second sens sont configurées à la manière de rigoles ouvertes vers le bas.

4. Couche de revêtement perméable aux liquides selon l'une quelconque des revendications précédentes, caractérisée en ce que les côtés (6) des côtes (2) de premier sens présentent des bords inférieurs libres (6A) le long desquels les côtes (3) de second sens sont rattachées par leur face supérieure (7) aux côtes (2) de premier sens.

5. Couche de revêtement perméable aux liquides selon l'une quelconque des revendications 1 à 3, caractérisée en ce que les côtes (3) de second sens passent entre deux côtes (2) de premier sens voisines, depuis un bord inférieur libre (6A) du côté de l'une des deux côtes (2) de premier sens voisines qui se trouve en vis-à-vis de l'autre des deux côtes (2) de premier sens voisines, vers l'autre zone de la face (6) opposée de l'autre des deux côtes (2) de premier sens voisines.

6. Couche de revêtement perméable aux liquides selon l'une quelconque des revendications précédentes, caractérisée en ce qu'entre deux côtes (2) de premier sens voisines, un canal (10) est chaque fois formé dans le premier sens.

7. Couche de revêtement perméable aux liquides selon la revendication 6, caractérisée en ce que le canal (10) présente, sur son fond, les côtes (3) de second sens et les ajours (4).

8. Couche de revêtement perméable aux liquides selon l'une quelconque des revendications précédentes, caractérisée en ce que les côtes (3) de second sens présentent une épaisseur inférieure à celle des côtes (2) de premier sens.

9. Couche de revêtement perméable aux liquides selon l'une quelconque des revendications précédentes, caractérisée en ce que la couche de revêtement (1) est formée par un procédé de formage thermique d'une feuille thermoplastique.

10. Couche de revêtement perméable aux liquides selon l'une quelconque des revendications précédentes, caractérisée en ce que la couche de revêtement (1) est hydrophobe et dotée d'un principe actif hydrophile.
